# EUROPEAN PATENT APPLICATION

(11) **EP 1 255 112 A2**
(43) Date of publication of application: **06.11.2002**
(21) Application number: 02253019.0
(22) Date of filing: 29.04.2002
(51) Int. Cl.: G01N 33/68, G01N 33/566

(54) **Treatment of T-cell mediated diseases**

(30) Priority: 30.04.2001 US 287511 P
(71) Applicant: Pfizer Products Inc., Groton, Connecticut 06340 (US)
(72) Inventor: Gladue, Ronald P., Pfizer Global Research, Groton, Connecticut 06340 (US); Martin, William Holt, Pfizer Global Research, Groton, Connecticut 06340 (US); Poss, Christopher Stanley, Pfizer Global Research, Groton, Connecticut 06340 (US)
(74) Representative: Hayles, James Richard

(57) **Abstract**

The present invention relates to the discovery that certain adverse inflammatory responses, allergic diseases, undesired immune responses including transplant rejection and autoimmune disease, and other disease states, can be treated or prevented by modulating the binding of the particular chemokines SLC (secondary lymphoid chemokine), and MIP-3β (macrophage inflammatory protein-3b), to immune system T cells and dendritic cells. The present invention is also related to methods for screening for therapeutic compounds useful in the treatment of SLC mediated and MIP-3β mediated disorders, and to the compounds detected by such screens. Appropriate assay methodology is also disclosed.

## Description

### Field of the Invention

The present invention relates to therapeutic modulation of T-cell mediated cellular processes. T-cells ("thymus-derived" cells) are responsible for numerous cell-mediated immune functions, and indirectly, by stimulating B-cells, contribute to antibody production. The cell membrane of a T-cell contains numerous receptor and accessory protein molecules that facilitate activation of T-cells, differentiation of T-cells into various subtypes, and the interaction of T-cells with other cells or cell components, including the migration of T-cells to disease sites.

Although T-cell mediated responses are normally of great benefit, there are circumstances where it is appropriate to suppress or otherwise modulate immune response mediated by T-cells. An important example is organ, tissue, or cell transplantation, where suppression of immune response against the transplanted cells is essential. Additional examples include treatment of allergy, autoimmune disease, and disease states involving inflammation. Since T-cells are involved in so many immune-mediated processes, and such processes generally involve overlapping use of various T-cell proteins and signaling functions, it has been very difficult to target only certain T-cell functions, without adversely affecting other desirable cellular processes.

T-cells differentiate and proliferate in response to recognition of antigens (generally, foreign macromolecules, or smaller molecules complexed with self macromolecules) in order to carry out various cell-mediated immune processes. Recognition of antigen, followed by functional and morphological changes in the T-cell, results in T-cell activation. Among the functions carried out by differentiated T-cells are (1) killing of virus-infected self cells, (2) killing foreign cells, (3) activation of cells (for example, macrophages) that are capable of engulfing foreign particles (such as bacteria and viruses), and in turn processing their macromolecules for presentation to, and activation of, additional T-cells, (4) suppression of immune response of B-cells and T-cells to antigen, which, for example, may act to establish immune tolerance, (5) activation of other T-cells, and (6) once themselves activated by antigen, helping B-cells respond to foreign antigens so that antibodies can be produced. In some cases these effects are carried out by direct contact of T-cells with their targets, and in other cases T-cells secrete a variety of substances (generically termed lymphokines) in order to activate additional target cells at a distance, or both mechanisms may be involved.

As will be mentioned in greater detail below, the combined presence of all of these interrelated systems can also lead to disadvantageous effects. For example, the immune system may react aginst a self-macromolecule leading to autoimmune disease. Additionally, release of some lymphokines (such as γ-interferon) by T-cells may cause macrophage cells to not only migrate to a site of infection or tissue damage, but to release other soluble factors that slowly trigger undesired inflammation (for example, in delayed type hypersensitivity).

Inflammatory responses in the body are representative of circumstances where prevention of participation by immune system components is sometimes of great value. Generally speaking, the inflammatory response is a protective mechanism that facilitates response to local injury. For example, leakage of tissue fluids into the affected area facilitates contact with antibodies, and also permits the migration of white blood cells to directly combat any injurious agent. Unfortunately, an inflammatory response may be inappropriate, that is, it may continue for an excessive period of time or involve participation by inflammatory system components that, unfortunately, act to damage to the body, thereby contributing to, or even defining, a disease state. Allergies and asthma represent a major class of complex, and typically chronic, inflammatory disease. As is typical of many inflammatory disease states, allergic disease states also involve aberrant or otherwise undersired activation of the immune system. Accordingly, there are numerous circumstances where it is medically appropriate to interefere with inflammatory processes.

As previously mentioned, there are additional circumstances where it is desireable to suppress normal immune system function, such as following transplant of cells, tissues or organs. However, given the complex nature of the immune system processes, it has proved difficult to identify effective and safe methods to suppress normal immune function.

As aforementioned, autoimmune disease, transplant rejection, allergy, and inflammation represent disease states wherein undesired activation of antigen-specific T-cells appears necessary for induction and/or progression of the unwanted clinical state. Accordingly, pharmaceutical compounds that interrupt activation of T-cells, or specific downstream signalling events, are expected to be of great therapeutic value. See, for example, J. H. Hanke, et al., *Inflammation Research,* 44, pp. 357-371, 1995.

T-cells recognize antigen through membrane glycoprotein receptors, called TcR, which are, in part, similar in structure and sequence to the antibodies of B-cells. The genetic elements from which the two proteins classes are expressed are undoubtedly of common origin. In general, T-cells only recognize antigen that is presented to them, in processed form, on the surface of other cells. Like antibody producing B-cells, each individual progenitor T-cell only recognizes a particular amino acid or carbohydrate sequence and/or other molecular structure (termed an epitope) in the processed antigen, which structure is usually unqiue to the antigen . Such specific recognition permits response against a wide range of foreign macromolecules, and is a necessary feature of mechanisms whereby immune responses against self-molecules are normally prevented.

Following the binding of antigen to the T-cell surface, numerous events must occur in the cell membrane and inside the T-cell to complete its activation. As reviewed in *Hanke et* *al.*, activation of the T-cell involves association of other cell membane glycoproteins, such as among CD4, CD8, CD3 and CD28, with the TcR, and also phosphorylation of tyrosine amino acid residues in these proteins (see C.H. June et al., *Journal of Immunology,* 144, pp. 1591-1599 (1990), and D. B. Strauss et al., *Cell,* 70, pp. 585-593, 1992).

The present invention is directed to preventing undesired immune responses. According to the practice of the invention, naive T-cells (T cells that have never been exposed to their cognate antigen) are prevented from contacting their target antigens, and thus do not undergo clonal expansion, and differentiation into effector and memory T-cells.

### Reported Developments

Recently, further progress has been made in understanding how individual T-cells first come into contact with the specific antigens that they are capable of recognizing. In one such mechanism, certain white blood cells known as dendritic cells capture antigen and process it, as a complex with proteins of the cell membrane, for presentation on their cell surfaces. Dendritic cells are believed to be the most potent type of such antigen presenting cells (APCs).

Leukocytes (including dendritic cells and lymphoctyes) normally recirculate between the peripheral blood vessels and the lymphatic system. Following recognition and capture of antigen, dendritic cells then migrate via the lymphatic system into secondary lymphoid organs (for example, lymph nodes, tonsils, Peyer's patches, and spleen) where they become localized in specialized zones, termed T cell zones. At the same time, naive T cells continuously migrate to the secondary lymphoid organs. Using specialized mechanisms of attachment, naive T-cells also penetrate into the T cell zones (similar mechanisms permit T cells to adhere to the walls of blood vessel endothelium at sites of inflammation). T cells are then able to enter gaps between the endothelial cells, thus entering the tissue where they may follow chemotactic gradients. See M. Gunn et al., The Journal of Experimental Medicine, 189(3), pp. 451-460, 1999, for a review of these processes.

In the T-cell zones, the continuous flow of naive T cells is able to contact the wide range of antigens presented on the now localized dendritic cells. Although most of the naive T-cells return to circulation, those that encounter their particular cognate antigen are retained in the T-cell zones, where they undergo clonal expansion, and differentiate into effector and memory T-cells (see, for example, M. Gunn et al., at 452)

It has been determined that the localization of both dendritic cells and naive T cells to the secondary lymphoid organs, and the T cell zones thereof, is mediated by chemokines (low molecular weight hormone-like proteins) which trigger this chemotactic activity. In a similar fashion, certain chemokines also direct dendritic cells and T cells to sites of inflammation.

In particular, SLC (secondary lymphoid organ chemokine) appears to be required for migration of both naive T cells and antigen-carrying dendritic cells into T cell zones where antigen presentation to the T cells occurs. See M. Gunn et al., The Journal of Experimental Medicine, 189(3), pp. 451-460, 1999, and T. Engeman et al., The Journal of Immunology, 164, pp.5207-5214, 2000. The chemokine MIP-3β has also been determined to contribute to lymphocyte migration, and infiltration of target tissues. Both of these chemokines exert their effects at the CCR7 receptor which is present on T cells and dendritic cells. Both chemokines also have potent effects upon cell adherence during lymphocyte migration. See R. Yoshida et al., Journal of Biological Chemistry, 272, pp. 7118-7122, 1998 and J.J. Campbell et al., J. Cell Biology, 141, 1053-1059, 1998.

CCR7, also termed chemokine (C-C motif) receptor 7, is a transmembrane receptor protein whose cytoplasmic effects are mediated by cyclic GMP (therefore termed G-coupled) following binding of an appropriate ligand. CCR7 is present on naive and activated T cells, B cells, and dendritic cells , but is absent from other leukocytes such as neutrophils, monocytes and NK cells. Accordingly, an agent that interferes with CCR7 function should specifically inhibit migration of T-lymphocytes and dendritic cells to sites where T cells can become activated. Further, Ngo et al. (The Journal of Experimental Medicine, 188, 181-191, 1998) have demonstrated the apparent existence of a self amplifying feedback loop whereinby dendritic cells themselves release MIP-3β, potentially attracting naive T cells into contact with dendritic cells.

The present invention is directed to the development of pharmaceutical compounds that interfere with the normal binding of SLC and MIP-3β to dendritic cells and T cells via CCR7 receptor. The present invention is thus directed to therapeutic compounds that selectively interefere with certain intracellular and intercellular signaling events, including chemotactic responses of cells, thus permitting selective regulation of immune response.

### Summary of the Invention

The present invention is directed to methods for screening for therapeutic compounds that are useful in the treatment of disorders mediated by the interaction of SLC and CCR7. The present invention is also directed to methods for screening for therapeutic compounds that are useful in the treatment of disorders mediated by the interaction of MIP-3β and CCR7.

In various embodiments, the invention provides primary screening assays to identify modulators, that is, compounds that are antagonists and/or agonists, of SLC/CCR7 and MIP-3β/CCR7 interactions. These primary assays are adaptable to high-throughput screening.

In other embodiments, the invention provides secondary assays to further characterize the biological activity of such modulators and their resultant utilities. Certain identified compounds will be useful in the treatment and prevention of disorders and conditions in which CCR7 participates, such as, for example, allergy, asthma, inflammatory conditions generally, transplant rejection and automimmune disease. Certain identified compounds will be useful in the treatment and prevention of disorders and conditions where it is advantageous to enhance the interaction of either SLC or MIP-3β with CCR7.

The invention provides a method for identifying a compound that modulates an SLC or MIP-3β mediated process, comprising: a) contacting CCR7 with a test compound, in the presence of absence of SLC or MIP-3β; and b) determining the biological effects thereof. In a typical assay, the test compound will bind, and have effects, at the same site on CCR7 at which SLC or MIP-3β normally binds, although the skilled practitioner will recognize that this need not always be so, that is, the methodologies of the present invention may also be used to identify compounds acting at sites on CCR7 remote to the normal SLC and MIP-3β binding site.

In one embodiment, the compound identified is an antagonist which interferes with the interaction of SLC or MIP-3β with CCR7, or the normal result thereof. In a further embodiment, the compound identified is an agonist which mimics the normal effects of binding of SLC or MIP-3β at CCR7, but at an enhanced level. In a typical embodiment, the CCR7 activity that is measured is the ability to interact with SLC or MIP-3β. In additional embodiments of the invention, the test compound is an antibody specific for CCR7, or is an antibody specific for an epitope provided by SLC or MIP-3β.

The invention further provides a method for identifying a compound that modulates an SLC/CCR7 or MIP-3β/CCR7-mediated process comprising: a) contacting a CCR7-expressing cell with a test compound; and b) measuring the resultant level of a CCR7 activity, or the level of expression of CCR7 in the cell, such that if said level of measured activity or expression differs from that measured in the absence of the test compound, then a compound that modulates a SLC-CCR7 or MIP-3β-CCR7-mediated process is identified. In one embodiment, the CCR7 activity measured is the ability to interact with SLC. In a further embodiment, the CCR7 activity measured is the ability to interact with MIP-3β. In another embodiment, the CCR7 activity measured is the chemotactic response of T-cells to SLC or MIP-3β. In a less preferred embodiment, membrane vesicles derived from cells that express CCR7 can also be used, as can synthetic or reconstituted membrane preparations.

Also encompassed by the invention is a method for identifying a compound that modulates the binding of SLC (or MIP-3β) to CCR7, comprising: (a) contacting the CCR7 with SLC or MIP-3β (or an analog thereof) in the presence of a test compound; and (b) measuring the amount of SLC or MIP-3β (or analog) that is bound to CCR7, such that if the amount of bound SLC or MIP-3β measured in (b) differs from the amount of bound SLC or MIP-3β measured in the absence of the test compound, then a compound that modulates the binding of SLC or MIP-3β to CCR7 is identified. In yet another embodiment, the amount of bound SLC or MIP-3β is measured by contacting the cell with an SLC or MIP-3β specific antibody.

In still another embodiment, the SLC or MIP-3β is labeled and the amount of bound SLC or MIP-3β is measured by detecting the label. In one embodiment of this method, the SLC or MIP-3β is labeled with a fluorescent label or a radioactive label.

The invention further provides a method for detecting a CCR7 related disorder in a mammal comprising measuring either the level of CCR7 gene expression, or of CCR7 receptor, in a patient sample, such that if the measured level differs from the level found in clinically normal individuals, then a CCR7 related disorder may be present.

Also encompassed by the present invention are kits. A kit is provided comprising (a) SLC or MIP-3β, or an analog thereof, or (b) a CCR7 polypeptide, or (c) nucleic acid encoding a CCR7 polypeptide or, for example, (d) a cell expressing CCR7, as packaged in a container with appropriate instructions and additional reagents. In one embodiment, the kit further comprises instructions for use in detecting the presence of a CCR7-related disorder in a patient.

Accordingly, the present invention provides a method for detecting if a test compound affects the binding of secondary lymphoid chemokine (SLC) or macrophage inflammatory protein-3β (MIP-3β) to CCR7 receptor comprising:
(a) contacting a sample of CCR7 with SLC or MIP-3β and measuring the binding of SLC or MIP-3β thereto;
(b) contacting a similar sample of CCR7 with SLC or MIP-3β and also an amount of said test compound, and measuring the binding of said SLC or MIP-3β to said CCR7; and
(c) comparing the results in (a) and (b) to determine if the binding of SLC or MIP-3β is affected by the presence of said test compound.

The invention further provides a method for determining if a test compound affects an activity of CCR7 receptor that is normally dependent upon the binding of SLC or MIP-3β thereto, comprising the steps of:
(a) contacting a sample of CCR7 with SLC or MIP-3β and measuring a resultant activity of said CCR7;
(b) contacting a similar sample of CCR7 with SLC or MIP-3β in the presence of an amount of said compound, and measuring said resultant activity of CCR7; and
(c) comparing the results in (a) and (b) to determine if the SLC-dependent or MIP-3β-dependent activity of CCR7 is affected by the presence of said compound.

In preferred examples of the methods of the invention, the test compound is identified as an antagonist that interferes with the SLC dependent or MIP-3β dependent activity of CCR7. In an additional example of the invention, the test compound is an agonist that enhances the SLC dependent or MIP-3β dependent activity of CCR7.

In further preferred examples, the test compound is an antibody specific for CCR7, or is specific for for SLC or MIP-3β.

In further preferred examples of the methods of the invention, wherein the CCR7 receptor is present on the surface of a mammalian cell, the SLC dependent or MIP-3β dependent activity of CCR7 is an effect on the cell which may be selected from the group consisting of migration to an inflammatory site, migration to a site of antigen presentation, cell activation, cell proliferation, and secretion of a cytokine.

According to the practice of the invention, the SLC dependent or MIP-3β dependent activity of CCR7 contributes to a disease state that is selected from the group consisting of: an autoimmune disease, an inflammatory disease, an allergic disease, transplant rejection, reperfusion injury, atherosclerosis, restinosis, enhanced HIV infectivity mediated by co-receptor usage, a granulomatous disease, inflammation-associated infection, and metastasis of cancer cells. The course of such diseases can be treated according to the practice of the invention.

Automimmune diseases that can be treated according to the practice of the present invention include, without limitation, rheumatoid arthritis, type I diabetes (recent onset), lupus, inflammatory bowel disease, primary sclerosing cholangitis, optic neuritis, psoriasis, multiple sclerosis, polymyalgia rheumatica, uveitis, and vasculitis.

Acute and chronic inflammatory diseases that can be treated according to the practice of the present invention include, without limitation, osteoarthritis, adult respiratory distress syndrome, respiratory distress syndrome of infancy, ischemia reperfusion injury, and glomerulonephritis.

Allergic diseases that can be treated according to the practice of the present invention include, without limitation, asthma, allergic rhinitis, and atopic dermatitis.

Circumstances of transplant rejection that can be treated according to the practice of the present invention include, without limitation, xeno-transplants and transplants of human cells, tissues and organs whether rejection thereof is chronic or acute.

Granulomatous diseases that can be treated according to the practice of the present invention include, without limitation, sarcoidosis, leprosy, and tuberculosis.

Circumstances of inflammation associated with infection that can be treated according to the practice of the present invention include, without limitation, inflammation associated with hepatitis, influenza and Guillan-Barre syndrome, and viral inflammations generally.

Additional diseases that can be treated according to the practice of the invention include atherosclerosis, restinosis (including but not limited to restinosis following balloon and/or stent insertion), and enhanced HIV infectivity mediated by co-receptor usage.

Additional disease states than can be treated according to the practice of the invention include chronic bronchitis and cancer metastasis.

The compounds developed according to the practice of the present invention may also limit the production of cytokines at inflammatory sites, including but not limited to TNF and IL-1, as a consequence of decreasing dendritic cell-derived release of IL-12; and therefore provide benefit for diseases linked to TNF and IL-1 including, without limitation, congestive heart failure, pulmonary emphysema and dyspnea associated therewith, emphysema; HIV-1, HIV-2, HIV-3; cytomegalovirus (CMV) infection, adenovirus infection, infection with Herpes viruses (*Herpes zoster* and *Herpes simplex).* The compounds may also provide benefit for the sequelae associated with infection where such infection induces production of detrimental inflammatory cytokines such as TNF e.g., in circumstances of fungal meningitis, joint tissue damage, hyperplasia, pannus formation and bone resorption, psoriatic arthritis, hepatic failure, bacterial meningitis, Kawasaki syndrome, myocardial infarction, acute liver failure, lyme disease, septic shock, cancer, trauma, and malaria, etc.

The present invention also includes methods for diagnosing a disorder in a patient that is mediated by the interaction of CCR7 receptor and SLC or MIP-3β, comprising the steps of:
(a₁) measuring the level of CCR7 gene expression in a patient sample; or
(a₂) measuring an SLC dependent or MIP-3β dependent activity of CCR7 receptor in a patient sample; and
(b) comparing said measurement to that determined from clinically normal individuals.

Accordingly, there are also provided diagnostic kits, packaged in a container, comprising:
(a) SLC, or a fragment thereof; and
(b) a reagent selected from:
   (b₁) CCR7 protein or a fragment thereof,
   (b₂) nucleic acid encoding a CCR7 protein or a fragment thereof, and (b₃) cells expressing CCR7;
wherein such kits may further comprise instructions for use in detecting or treating a disorder in a patient that is mediated by the interaction of CCR7 receptor and SLC. Similar kits are provided wherein MIP-3β replaces SLC.

The terms "SLC mediated" and "MIP-3β mediated" as used herein include processes that are dependent and/or responsive, either directly or indirectly, to the level of expression, synthesis of, and/or activity of SLC or MIP-3β. Such processes include, but are not limited to allergic, asthmatic, and inflammatory processes, such as chemotaxis of inflammatory cells. The terms "SLC/CCR7-related disorder" and "MIP-3β/CCR7-related disorders", or conditions, and the like, as used herein refer to disorders and conditions in which SLC or MIP-3β participates, or which may be affected, beneficially or adversely, by the concentration of SLC or MIP-3β in a patient. Such disorders and conditions may result, for example, from an aberrant level of SLC or MIP-3β expression, synthesis and/or activity relative to levels found in normal, unaffected, unimpaired individuals. Such disorders include, but are not limited to, allergic disorders, asthmatic disorders, and inflammatory disorders, such as allergic rhinitis, allergenic asthma, bronchoconstriction, and autoimmune disorders.

For the purposes of the present invention, an "analog" of SLC or MIP-3β is a compound generally having one or more in-common structural features with SLC or MIP-3β, and which generally has the same general biological effect as these chemokines, at least under one condition of assay. Thus, in an assay, for example, in which the activity of potential SLC-agonist or SLC-antagonist compounds will be measured, the analog may be substituted for any SLC or MIP-3β itself that would otherwise be used in said assay.

### Brief Description of the Drawings

Figure 1 shows generally steps involved in leukocyte migration into tissues.

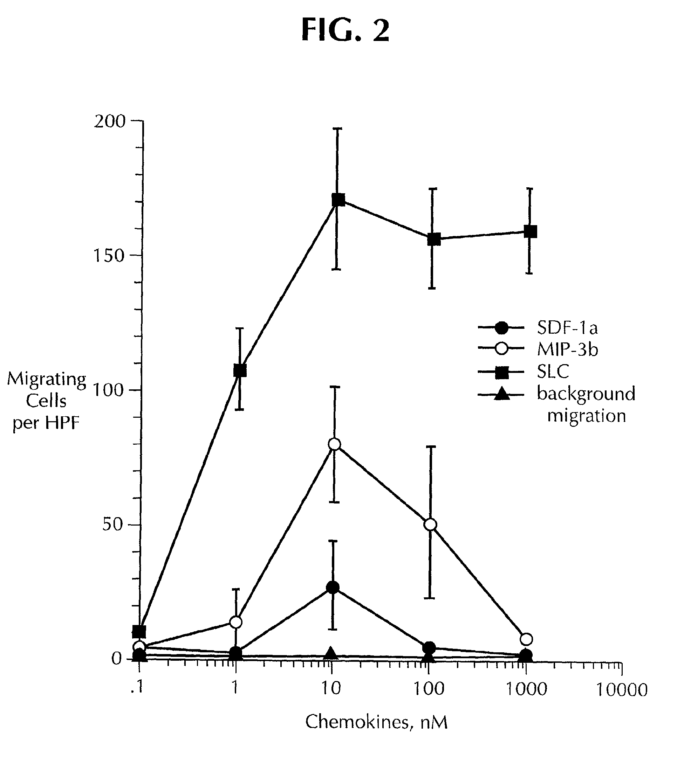

Figure 2 shows that SLC and MIP-3β are potent chemoattractants for Hut-78 cells.

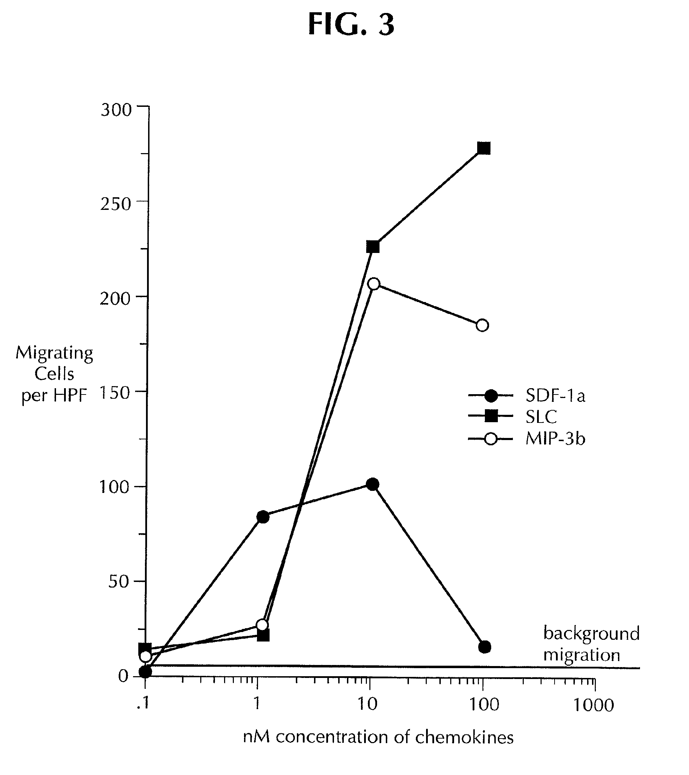

Figure 3 shows that SLC and MIP-3β are potent chemoattractants for PHA-activated human T cells.

### Detailed Description of the Invention

Disclosed herein are screening assays for the identification of therapeutic compounds useful for the detection and treatment of SLC and MIP-3β mediated disorders, such as allergic disorders, asthma, immune disorders and inflammatory response disorders. The assays are also adaptable for the detection of compounds that, relative to SLC and MIP-3β themselves, show enhanced binding and activity at CCR7. Such screening assays include first, primary screening assays, which may be adapted to high-throughput screening formats, and, second, secondary screening assays, which can be used to further characterize lead compounds identified in the primary screens. In addition, there are disclosed diagnostic and therapeutic methods for the use of identified compounds in the treatment of SLC and MIP-3β-related disorders.

The screening assays described herein may be used to identify, for example, organic compounds, peptides and proteins (including antibodies) that modulate the interaction of SLC or MIP-3β with CCR7.

Such identified compounds, and the like, may be used as agonists or antagonists of the chemotactic induction of T cells via CCR7 by SLC or MIP-3β. Compounds that bind to CCR7, thereby inhibiting binding of SLC or MIP-3β, and that either block chemotactic induction (antagonists) or enhance chemotactic induction (agonists) are useful according to the practice of the invention.

The methodology of the present invention is also useful to detect (or confirm the activity of) compounds that bind to SLC or MIP-3β, thereby preventing or enhancing binding of said SLC or MIP-3β to CCR7. The therapeutic activity of antibodies may be confirmed according to this aspect of the invention.

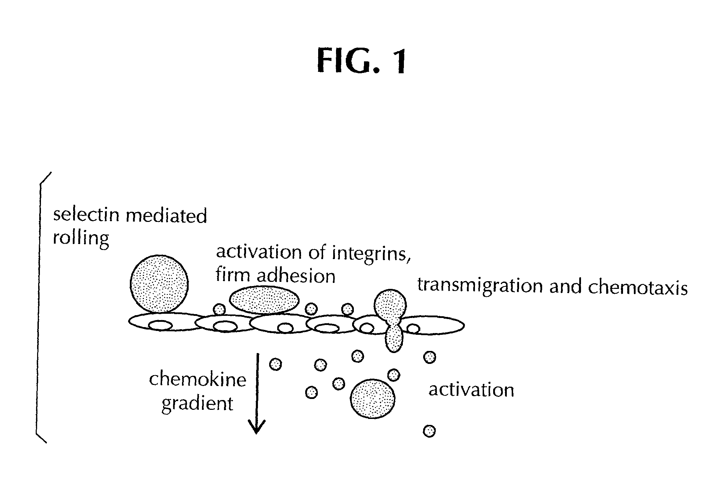

CCR7 is also know to play an important role in controlling integrin avidity and firm adherence of T lymphocytes. Lymphocyte recruitment into the secondary lymphoid tissues as well as into inflammatory sites is under the control of the high endothelial venules (HEV). The interaction of lymphocytes with the HEV and their migration into tissues involves several steps (see Figure 1 below). First, lymphocytes reversibly attach to endothelial cells via selectins in a process referred to as rolling. Cells next become firmly adherent to the endothelium in order to withstand shear forces caused by blood flow. This firm adherence step depends on integrins present on the cell surface and is thought to be controlled through G protein-coupled receptors ("GPCR") through the Giα subunit. Following firm adherence, cells move through junctions in endothelial cells then undergo chemotaxis into tissues, a process also shown to be G-protein dependent.

Chemokines are 8-10,000 MW chemotactic cytokines which exert their effects through GPCRs having seven transmembrane domains. Numerous studies have provided important evidence for a role for chemokines during the last step of tissue emigration (C.R. Mackay et al., Current Biology, 7, R384-386, 1997, and M. Baggiolini et al., Ann. Rev. Immunology, 15, pp. 675-705, 1997). This is further supported by observations concering the potent *in vitro* and *in vivo* chemotactic activity of chemokines (for example L.A. Beck et al., J. Immunology, 159, pp. 2962-2972, 1997), and the expression of chemokines at sites of inflammation (see for example, D.H. Adams et al., Transplantation, 61, pp. 817-825, 1996, and A.E. Koch et al., J. Clin, Invest., 93, pp.921-928, 1994). Further support is derived from observations concerning the ability of anti-chemokine neutralizing antibodies to modulate disease severity and tissue infiltration in animal models (see for example, W.J. Karpus et al., J. Immunol. ,155, pp. 5003-5010, 1995).

However, the identity of the GPCRs responsible for the initial "firm adhesion" step of lymphocyte migration has remained unknown. Most chemokines have minimal effects on this step of lymphocyte migration. Recently, three new chemokines have been shown to enhance integrin avidity and have potent effects on cell adherence under both static and flow conditions. These chemokines are SDF-1 α which interacts with the receptor CXCR4, and the chemokines MIP-3βand SLC, both of which interact with the receptor CCR7 (see R. Yoshida et al., Journal of Biological Chemistry, 272, pp.13803-13809, 1997; and J.J. Campbell et al., Science, 279, pp.381-384, 1998).

Experiments with SLC have demonstrated its ability to trigger rapid integrindependent arrest of T-lymphocytes under both static and flow conditions. As little as a 1 second exposure of lymphocytes to SLC will promote adherence to MadCAM and ICAM-1 proteins (M.D. Gunn et al., Keystone Conference, Lake Tahoe, Nevada, March 22, 1998, and J.J. Campbell et al., Science, 279, pp. 381-384, 1998). This increase in adherence is not related to an increase in integrin expression but rather a change in integrin avidity shown to be necessary to cause firm adherence of cells. A role for the receptor CCR7 was confirmed in these studies by using CCR7 transfected cells.

In support of these *in vitro* observations, the important role of SLC in T cell adherence and tissue emigration *in vivo* has been recently suggested. A strain of mouse, known as the DDD/1 strain, has been characterized as having a defect in T cell homing and a concomitant reduction in lymph node size characterized by a 80% reduction in T lymphocyte numbers in the lymph nodes (H. Nakano et al., Eur. J. Immunol., 27, 215-221, 1997). In addition, these animals have an increase in the number of circulating T-lymphocytes in the peripheral blood. Experiments have demonstrated that this defect in lymphocyte trafficking was not at the level of the T cells, since transfer of these cells into a syngeneic animal resulted in normal homing. Rather, the defect appeared to be due to a diminished ability of secondary lymphoid tissue to support the entry of T cells. In unpublished observations reported at several meetings by Dr. M. Gunn (UCSF), using in situ hybridization, Dr. Gunn demonstrated that peripheral lymph nodes (HEV) in these animals are completely devoid of SLC whereas wild-type mice have a high expression of SLC in the HEV. This is consistant with recent linkage analysis localizing the autosomal recessive defect in DDD/1 mice on chromosome 4 in a region that contains a cluster of chemokine genes. The phenotype of these animals is consistent with a defect in lymphocyte firm adherence.

### CCR7 promotes the chemotaxis of lymphocytes and dendritic cells

Although the ability of SLC to promote lymphocyte firm adherence alone would suggest that preventing interaction with its receptor, CCR7, by a pharmacological agent would decrease T cell inflammation and prevent transplant rejection, CCR7 has additional properties that further strengthen it's potential as a drug target. At the recent chemokine Gordon conference, Gunn et. al. indicated a role for CCR7 and its ligands SLC and MIP-3β in dendritic cell migration *in vitro* and *in vivo*, suggesting a critical role for CCR7 in controlling antigen specific responses. Gunn demonstrated that dendritic cell migration was suppressed in the SLC-deficient mouse, DDD/1. Further, studies have now demonstrated that the kinetics of CCR7 expression by dendritic cells is such that once the cells enter lymphoid tissues, they may be retained, potentially maximizing their ability to present antigen (S. Sozzani et al., J. Immunology, 161, pp.1083-1086, 1998).

A second property of CCR7 is its role in lymphocyte chemotaxis. We have examined the effects of both SLC and MIP-3β on Hut 78 and primary T cells and compared their activity to other chemokines. Most chemokines tested have minimal effects on T cell chemotaxis (e.g. MIP-1α, MIP-1β, RANTES, MCP-1, MCP-3, Tarc, and IP-10). In contrast, SLC and MIP-3β are the most potent T cell chemotactic agents that we have examined, outperforming even SDF-1α. Since CCR7 has been shown to be present on both naive and activated T cells as well as B cells but is absent on monocytes, NK cells, and neutrophils, an agent that interferes with CCR7 should specifically block lymphocyte and dendritic cell migration. As aforementioned, data published recently by V.N. Ngo et al., J. Exp. Med., 188, pp. 181-191, 1998, suggests an amplification loop where dendritic cells themselves can release MIP-3β, potentially attracting T cells into contact with dendritic cells.

### Screening Strategy

A useful screening strategy is as follows. A radioligand receptor binding assay is utilized using either Hut 78 cells or CCR7 transfected cells. Initial binding experiments are performed with radiolabeled MIP-3β. Once "hits" are identified, the ability of compounds to inhibit the chemotaxis of Hut-78 and primary T cells in response to MIP-3β and SLC are examined. Specificity against other chemokine receptors utilizing ligand binding and chemotaxis assays is also determined. Activity in animal models of transplantation is next performed for final candidate identification. To support the determination of biochemical efficacy in Phase I trials, CCR7 ligand (MIP-3β or SLC) is injected into the skin of normal volunteers to assess cell infiltration. This human model system has been used in the CCR1 approach and will be useful to establish a pharmacodynamic end-point in phase I trials, prior to Phase II transplant studies for CCR7 studies.

Compounds that affect CCR7 gene expression including molecules ( e.g., proteins or small organic molecules) that affect transcription, or interfere with splicing events so that expression of the full length or the truncated form of CCR7 can be modulated, can also be identified in the screens of the invention.

Further, it should be noted that the assays described can also identify compounds that modulate CCR7 signal transduction (*e.g.,* compounds which affect downstream signaling events, such as inhibitors or enhancers of G-protein activities which may participate in transducing the signal activated by SLC or MIP-3β, or other ligand binding to CCR7. The identification and use of such compounds which affect signaling events downstream of CCR7 and thus modulate effects of CCR7 on allergic or inflammatory responses, for example, are within the scope of the invention.

The primary screening assays described herein are designed to detect compounds that modulate the SLC or MIP-3β/CCR7 interaction, that is, the test compounds act in the place of SLC or MIP-3β at the CCR7 receptor, either negatively or positively compared with said SLC or MIP-3β, or which combine with or otherwise modify SLC (or MIP-3β), thereby affecting how it acts at CCR7. As described in detail below, such assays are functional assays, such as binding assays, that can be adapted to a high-throughput screening methodologies.

Binding assays may be performed either as direct binding assays or as competition binding assays. In a direct binding assay, a test compound is tested for binding either to the CCR7 receptor, or to ligand SLC or MIP-3β. Then, in a second step, the test compound is tested for its ability to modulate the interaction of either SLC or MIP-3β with CCR7 receptor. Competition binding assays, on the other hand, assess the ability of a test compound to compete with SLC or MIP-3β for binding to CCR7.

In a direct binding assay, either SLC or MIP-3β and/or CCR7 is contacted with a test compound under conditions that allow binding of the test compound to the ligand or the receptor. The binding may take place in solution or on a solid surface. Preferably, the test compound is previously labeled for detection. Any detectable group may be used for labeling, such as but not limited to, a luminescent, fluorescent, or radioactive isotope or group containing same, or a nonisotopic label, such as an enzyme or dye. After a period of incubation sufficient for binding to take place, the reaction is exposed to conditions and manipulations that remove excess or non-specifically bound test compound. Typically, this involves washing with an appropriate buffer. Finally, the presence of a ligand-test compound complex or a receptor-test compound complex is detected.

In a competition binding assay, test compounds are assayed for their ability to disrupt or enhance the binding of SLC or MIP-3β ligand to CCR7 receptor. Labeled SLC or MIP-3β may be mixed with CCR7 or a fragment or derivative thereof, and placed under conditions in which the interaction between them would normally occur, either with or without the addition of the test compound. The amount of labeled SLC or MIP-3β that binds CCR7 may be compared to the amount bound in the presence or absence of test compound.

An affinity binding assay may be performed using a CCR7 fragment which is immobilized to a solid support. Typically, the non-immobilized component of the binding reaction, in this case either SLC, MIP-3β, or the test compound, is labeled to enable detection. A variety of labeling methods are available and may be used, such as detection of luminescent, chromophoric, fluorescent, or radioactive isotopes or groups, or detection of nonisotopic labels, such as enzymes or dyes. In one preferred embodiment, the test compound is labeled with a fluorophore such as fluorescein isothiocyanate (FITC, available from Sigma Chemicals, St. Louis).

The labeled test compounds, or SLC or MIP-3β plus test compounds, are then allowed to contact with the solid support, under conditions that allow specific binding to occur. After the binding reaction has taken place, unbound and non-specifically bound test compounds are separated by means of washing the surface. Attachment of the binding partner to the solid phase can be accomplished in various ways known to those skilled in the art, including but not limited to chemical cross-linking, non-specific adhesion to a plastic surface, interaction with an antibody attached to the solid phase, interaction between a ligand attached to the binding partner (such as biotin) and a ligand-binding protein (such as avidin or streptavidin) attached to the solid phase, and the like.

Finally, the label remaining on the solid surface may be detected by any detection method known in the art. For example, if the test compound is labeled with a fluorophore, a fluorimeter may be used to detect complexes.

A labeled SLC or MIP-3β may be mixed with cells that express CCR7, or less preferably, mixed with crude extracts obtained from such cells, and the test compound may be added. Isolated membranes may be used to identify compounds that interact with CCR7. For example, in a typical experiment using isolated membranes, cells may be genetically engineered to express CCR7. Membranes can be harvested by standard techniques and used in an *in vitro* binding assay. Labeled ligand (*e.g*., ¹²⁵I-labeled SLC) is bound to the membranes and assayed for specific activity; and specific binding is determined by comparison with binding assays performed in the presence of excess unlabeled (cold) ligand. Alternatively, soluble CCR7 may be recombinantly expressed and utilized in non-cell based assays to identify compounds that bind to CCR7. The recombinantly expressed CCR7 polypeptide(s) or fusion proteins containing one or more of the extracellular domains of CCR7 can be used in the non-cell based screening assays. Alternatively, peptides corresponding to one or more of the cytoplasmic domains of CCR7, or fusion proteins containing one or more of the cytoplasmic domains of CCR7, can be used in non-cell based assay systems to identify compounds that bind to the cytoplasmic portion of the CCR7; such compounds may be useful to modulate the signal transduction pathway of the CCR7. In non-cell based assays, the recombinantly expressed CCR7 is attached to a solid substrate such as a test tube, microtitre well or a column, by means known to those in the art (see Ausubel *et al.,* supra). The test compounds are then assayed for their ability to bind to the CCR7.

In a further embodiment, for example, a phage library can be screened by passing phage from a continuous phage display library through a column containing purified CCR7, or derivative, analog, fragment, or domain, thereof, linked to a solid phase, such as plastic beads. By altering the stringency of the washing buffer, it is possible to enrich for phage that express peptides with high affinity for CCR7. Phage isolated from the column can be cloned and the affinities of the short peptides can be measured directly. Sequences for more than one oligonucleotide can be combined to test for even higher affinity binding to CCR7. Knowing which amino acid sequences confer the strongest binding to CCR7, computer models can be used to identify the molecular contacts between CCR7 and the test compound. This will allow the design of non-protein compounds which mimic those contacts. Such a compound may have the same activity of the peptide and can be used therapeutically, having the advantage of being efficient and less costly to produce.

In another specific embodiment of this aspect of the invention, the solid support is membranes containing CCR7 attached to a microtiter dish. Test compounds, for example, cells that express library members are cultivated under conditions that allow expression of the library members in the microtiter dish. Library members that bind to the protein (or nucleic acid or derivative) are harvested. Such methods, are described by way of example in Parmley & Smith, 1988, Gene 73:305-318; Fowlkes *et al.,* 1992, BioTechniques 13:422-427; PCT Publication No. WO 94/18318; and in other references cited herein.

In another embodiment of the present invention, interactions between CCR7 or SLC (or MIP-3β) and a test compound may be assayed *in vitro.* Known or unknown molecules are assayed for specific binding to CCR7 peptides, or derivatives, under conditions conducive to binding, and then molecules that specifically bind to CCR7 are identified. The two components can be measured in a variety of ways. One approach is to label one of the components with an easily detectable label, place it together with a test component(s) under conditions that allow binding to occur, perform a separation step which separates bound labeled component from unbound labeled component, and then measure the amount of bound component. In one embodiment, CCR7 can be labeled and added to a test agent, using conditions that allow binding to occur. Binding of the test agent can be determined using polyacrylamide gel analysis to compare complexes formed in the presence and absence of the test agent.

Additionally, binding of SLC (or MIP-3β) to CCR7 may be assayed in intact cells in animal models. A labeled SLC, for example, may be administered directly to an animal, with and without a test compound. The uptake of SLC may be measured in the presence and the absence of test compound. For these assays, host cells to which the test compound has been added may be genetically engineered to express the CCR7 and/orSLC (or MIP-3β), which may be transient, induced or constitutive, or stable. For the purposes of the screening methods of the present invention, a wide variety of host cells may be used including, but not limited to, tissue culture cells, mammalian cells, yeast cells, and bacteria. Each cell type has its own set of advantages. Mammalian cells such as T cells or other cells that express CCR7 may be a preferred cell type in which to carry out the assays of the present invention. Bacteria and yeast are relatively easy to cultivate but process proteins differently than mammalian cells.

In some cases, a functional CCR7 ligand may not form a thermodynamically stable complex with CCR7, and would therefore not be detectable by primary assays which require the formation of stable binary complex. Such ligands, however, may be detectable by kinetic measurements of complex formation. Such methods include, for example, kinetic measurement of on rates and off rates of ligand binding to the receptor. Thus, binding assays of the invention also include kinetic studies and measurements.

In addition, in some instances, responses of G protein-coupled receptors have been observed to subside, or become desensitized with prolonged exposure to ligand. In a further embodiment of the invention, assays may be utilized to identify compounds that block the desensitization of the CCR7 receptor, and such compounds may be used to sustain the activity of CCR7. Such compounds can be also used as part of a therapeutic method for the treatment of SLC or MIP-3β-related disorders, such as allergenic and inflammatory response disorders, such as asthma.

In one embodiment of the invention, for example, an assay which is less dependent upon the formation of thermodynamically stable complexes is a scintillation proximity assay (described in U.S. patent no. 4,568,649). Purified or partially purified CCR7 or CCR7-containing membranes are coated onto the surface of a scintillant-loaded solid phase (*e.g.,* beads) and the solid phase is treated with a blocking agent such as albumin or serum. Radiolabeled test compounds (*e.g.,* ³³P-labeled) are then mixed with the CCR7-coated beads, under conditions that would allow specific binding of a candidate test compound to the CCR7 on the solid phase. After washing to remove excess or non-specific binding, if specific binding of a labeled test compound and CCR7 took place, the radiolabel is brought into close proximity to the scintillant, allowing the scintillant to emit light, which is detectable with a scintillation counter.

In an alternative embodiment, an affinity capture scintillation proximity assay may be used so that binding may be performed in solution. In this assay, CCR7 is purified and labeled with an affinity label, such as biotin. Biotinylated CCR7 is then mixed with the radiolabeled test compound, under conditions that allow solution binding to occur. Biotinylated CCR7 including complexes of CCR7 and test compound are captured on streptavidin-coated scintillant-loaded beads (available from Amersham) and counted in a scintillation counter, as described above.

Chemotaxis assays may also be used as primary assays. One biological effect of the interaction between a receptor of type CCR7 and an attractant is the induction of the directional migration of cells expressing the receptor toward the particular attractant, a process known as chemotaxis. A chemotaxis assay, as described herein, may be used to screen compounds that interfere with the interaction of the CCR7 receptor and the attractant SLC or MIP-3β. Such chemotaxis assays are adaptable to high throughput screening methods, and can thus be used in as a primary assay to identify CCR7 antagonists. A number of techniques have been developed to assay chemotactic migration (see, *e.g.,* Leonard *et al.*, 1995, "Measurement of α and β Chemokines", in Current Protocols in Immunology, 6.12.1-6.12.28, Ed. Coligan *et al.,* John Wiley & Sons, Inc. 1995).

In one embodiment, for example, a compound can be tested for its ability to modulate the ability of SLC (or MIP-3β) to induce migration of cells that express CCR7 using a chemoattractant gradient in a multiwell Boyden chemotaxis chamber. This apparatus typically contains a chamber bottom with 48 U-bottom wells, into which is placed the chemoattractant, or compound to be tested for chemoattractant activity. A polycarbonate membrane covered with a sealing gasket separates the bottom chamber from the 48-holed chamber top, into which a cell suspension is added to the wells formed by the membrane and the chamber top (see Example 2).

In a specific example of this method, a competitive assay is performed to test compounds for their ability to interrupt the attraction of CCR7 cells to SLC (or MIP-3β). In this method, test compounds are diluted into the bottom wells of the Boyden chemotaxis chamber. A constant amount of SLC is also added to this dilution series, at a concentration known to have a chemotactic effect on CCR7 cells. As a control, at least one aliquot contains only SLC. Cells expressing CCR7 are resuspended, for example, at 3-3.5 x 10⁶ cells/ml in RPMI 1640 supplemented with 10 mM HEPES and 1 mg/ml bovine serum albumin (Sigma, St. Louis, MO), and placed into the upper wells of the chamber. The chambers are incubated for 90-120 minutes at 37°C in a humidified CO₂ incubator. After the incubation period, the number of migrating cells on the lower surface of the membrane filter is counted using light microscopy. The contribution of the test compound to the chemotactic activity of SLC is measured by comparing the chemotactic activity of the aliquots containing only SLC with the activity of aliquots containing test compound and SLC. If addition of the test compound to the SLC solution results in a decrease in the number of cells detected on the lower surface of the membrane relative to the number of cells detected using a solution containing only SLC, then there is identified an antagonist of SLC induction of chemotactic activity of cells expressing CCR7. In contrast, if the addition of the test compound to the SLC solution results in a decrease in the number of cells detected on the lower surface of the membrane (relative to the number of cells detected using a solution containing only SLC), then an agonist of SLC induction of chemotactic activity of cells expressing CCR7 is identified.

The methods of the invention can routinely be performed in a high-throughput fashion for rapidly screening multiple test compounds. In particular, the cell systems used in such methods can be expressed and assayed in any multiple copy format known to those of skill in the art, including, but not limited to microtiter plates, spotting on agar plates, agar wells, spotting on chips and the like. Likewise, standard multiple manipulation techniques including but not limited to robotic handling techniques, can be utilized for multiple deposition of cells and/or test compounds.

After identification of a test compound that modulates the interaction of SLC (or MIP-3β) with CCR7, secondary screening assays may be used to further characterize the test compound for its effect on the biological activity of SLC (or MIP-3β), CCR7, and CCR7-signaling pathways. Various assays can be adapted to use as a secondary screen. For example, such methods include, but are not limited to, binding assays, chemotaxis assays, adhesion assays, intracellular calcium mobilization assays, oxygen release assays, and actin polymerization assays. Examples of such assays are discussed in detail hereinbelow.

Binding assays may be performed as a secondary assay, instead of, or in addition to a primary binding assays. In one embodiment, where a direct binding assay was used as a primary screen, a competition assay may be used as a secondary screen. In another embodiment, a binding assay may be used for a compound identified by a functional primary screen, such as a high-throughput chemotaxis screening assay. In another embodiment, a compound identified in a primary screen using a binding assay may be further analyzed in a secondary screen using a second type of binding assay. For example, a compound identified using a CCR7 affinity column assay, may be tested in a secondary screen by kinetic analysis of its binding interaction.

The chemotaxis assay may be used as a secondary assay. A test compound [identified by the primary screening assay to interfere with the binding of SLC (or MIP-3β) to CCR7, or, alternatively, to enhance the binding of SLC (or MIP-3β) to CCR7], can be tested for biological activity using a chemotaxis assay. As described above, chemokines can induce directional migration of cells via their interaction with a cell-type specific chemokine receptor, and a number of techniques have been developed to test this chemotactic migration (see, *e.g.,* Leonard *et al.,* 1995, "Measurement of α and β Chemokines", in Current Protocols in Immunology, 6.12.1-6.12.28, Ed. Coligan *et al*., John Wiley & Sons, Inc. 1995). Thus, in one embodiment, for example, a compound can be tested for its ability to modulate the ability of SLC to induce migration of cells that express CCR7 using a chemokine gradient in a multiwell Boyden chemotaxis chamber.

In a specific example of this method, a serial dilution of a SLC/CCR7 antagonist or agonist test compound identified in the primary screen is placed in the bottom wells of the Boyden chemotaxis chamber. A constant amount of SLC is also added to this dilution series. As a control, at least one aliquot contains only SLC. The method and the assay conditions are as described in the primary screening assay, and the number of migrating cells on the lower surface of the membrane filter is counted using light microscopy. The contribution of the antagonist or agonist compound to the chemotactic activity of SLC is measured by comparing the chemotactic activity of the aliquots containing only SLC with the activity of aliquots containing test compound and SLC. If addition of the test compound to the SLC solution results in a decrease in the number of cells detected on the lower surface of the membrane relative to the number of cells detected using a solution containing only SLC, then an antagonist of SLC induction of chemotactic activity of cells expressing CCR7 is identified. In contrast, if the addition of the test compound to the SLC solution results in a decrease in the number of cells detected on the lower surface of the membrane relative to the number of cells detected using a solution containing only SLC then an agonist of SLC induction of chemotactic activity of cells expressing CCR7 is identified.

The screening assays described herein may be used to identify organic compounds, or peptides or proteins, for example, that modulate the interaction of SLC (or MIP-3β) with CCR7. The substances which may be screened in accordance with the invention therefore also include antibodies and fragments thereof. Peptidomimetic organic compounds that bind, for example, to the extra-cellular domain (ECD) of CCR7 either inhibit the activity triggered by the natural ligand *(i.e.,* antagonists) or mimic the activity triggered by the natural ligand (*i.e.*, agonists), may also be screened.

Compounds that may be used for screening include, but are not limited to, peptides such as, for example, soluble peptides, including but not limited to members of random peptide libraries; (see, *e.g.,* Lam *et al*., 1991, Nature 354:82-84; Houghten *et al*., 1991, Nature 354:84-86), and combinatorial chemistry-derived molecular library made of D- and/or L-configuration amino acids, phosphopeptides (including, but not limited to, members of random or partially degenerate, directed phosphopeptide libraries; see, *e.g.,* Songyang *et al.,* 1993, Cell 72:767-778), antibodies (including, but not limited to, polyclonal, monoclonal, humanized, anti-idiotypic, chimeric or single chain antibodies, and FAb, F(ab')₂ and FAb expression library fragments, and epitope-binding fragments thereof), and small organic or inorganic molecules.

Diversity libraries, such as random or combinatorial peptide or nonpeptide libraries can be screened for molecules that specifically bind to the CCR7 receptor. Many libraries are known in the art that can be used, *e.g.,* chemically synthesized libraries, recombinant (*e.g.*, phage display libraries), and *in vitro* translation-based libraries.

Examples of chemically synthesized libraries are described in Fodor *et al*., 1991, Science 251:767-773; Houghten *et al.*, 1991, Nature 354:84-86; Lam *et al*., 1991, Nature 354:82-84; Medynski, 1994, Bio/Technology 12:709-710; Gallop *et al*., 1994, J. Medicinal Chemistry 37(9):1233-1251; Ohlmeyer *et al*., 1993, Proc. Natl. Acad. Sci. USA 90:10922-10926; Erb *et al.,* 1994, Proc. Natl. Acad. Sci. USA 91:11422-11426; Houghten *et al*., 1992, Biotechniques 13:412; Jayawickreme *et al.*, 1994, Proc. Natl. Acad. Sci. USA 91:1614-1618; Salmon *et al.,* 1993, Proc. Natl. Acad. Sci. USA 90:11708-11712; PCT Publication No. WO 93/20242; and Brenner and Lerner, 1992, Proc. Natl. Acad. Sci. USA 89:5381-5383.

Examples of phage display libraries are described in Scott & Smith, 1990, Science 249:386-390; Devlin *et al.*, 1990, Science, 249:404-406; Christian, *et al.*, 1992, J. Mol. Biol. 227:711-718; Lenstra, 1992, J. Immunol. Meth. 152:149-157; Kay *et al.*, 1993, Gene 128:59-65; and PCT Publication No. WO 94/18318 dated August 18, 1994.

By way of examples of nonpeptide libraries, a benzodiazepine library (see *e.g*., Bunin *et al.*, 1994, Proc. Natl. Acad. Sci. USA 91:4708-4712) can be adapted for use. Peptoid libraries (Simon *et al.*, 1992, Proc. Natl. Acad. Sci. USA 89:9367-9371) can also be used. Another example of a library that can be used, in which the amide functionalities in peptides have been permethylated to generate a chemically transformed combinatorial library, is described by Ostresh et al. (1994, Proc. Natl. Acad. Sci. USA 91:11138-11142).

Screening the libraries can be accomplished by any of a variety of commonly known methods. See, *e.g.*, the following references, which disclose screening of peptide libraries: Parmley & Smith, 1989, Adv. Exp. Med. Biol. 251:215-218; Scott & Smith, 1990, Science 249:386-390; Fowlkes *et al., 1992;* BioTechniques 13:422-427; Oldenburg *et al.,* 1992, Proc. Natl. Acad. Sci. USA 89:5393-5397; Yu *et al*., 1994, Cell 76:933-945; Staudt *et al*., 1988, Science 241:577-580; Bock *et al*., 1992, Nature 355:564-566; Tuerk *et al*., 1992, Proc. Natl. Acad. Sci. USA 89:6988-6992; Ellington *et al*., 1992, Nature 355:850-852; U.S. Patent No. 5,096,815, U.S. Patent No. 5,223,409, and U.S. Patent No. 5,198,346, all to Ladner et al.; Rebar & Pabo, 1993, Science 263:671-673; and PCT Publication No. WO 94/18318.

Compounds that can be tested and identified methods described herein can include, but are not limited to, compounds obtained from any commercial source, including Aldrich (1001 West St. Paul Ave., Milwaukee, WI 53233), Sigma Chemical (P.O. Box 14508, St. Louis, MO 63178), Fluka Chemie AG (Industriestrasse 25, CH-9471 Buchs, Switzerland (Fluka Chemical Corp. 980 South 2nd Street, Ronkonkoma, NY 11779)), Eastman Chemical Company, Fine Chemicals (P.O Box 431, Kingsport, TN 37662), Boehringer Mannheim GmbH (Sandhofer Strasse 116, D-68298 Mannheim), Takasago (4 Volvo Drive, Rockleigh, NJ 07647), SST Corporation (635 Brighton Road, Clifton, NJ 07012), Ferro (111 West Irene Road, Zachary, LA 70791), Riedel-deHaen Aktiengesellschaft (P.O. Box D-30918, Seelze, Germany), PPG Industries Inc., Fine Chemicals (One PPG Place, 34th Floor, Pittsburgh, PA 15272). Further any kind of natural products may be screened using the methods of the invention, including microbial, fungal, plant or animal extracts.

Furthermore, diversity libraries of test compounds, including small molecule test compounds, may be utilized. For example, libraries may be commercially obtained from Specs and BioSpecs B.V. (Rijswijk, The Netherlands), Chembridge Corporation (San Diego, CA), Contract Service Company (Dolgoprudny, Moscow Region, Russia), Comgenex USA Inc. (Princeton, NJ), Maybridge Chemicals Ltd. (Cornwall PL34 OHW, United Kingdom), and Asinex (Moscow, Russia).

Still further, combinatorial library methods known in the art, can be utilize, including, but not limited to: biological libraries; spatially addressable parallel solid phase or solution phase libraries; synthetic library methods requiring deconvolution; the "one-bead one-compound" library method; and synthetic library methods using affinity chromatography selection. An example of the biological library approach preferably involves a peptide library, while the other four approaches are applicable to peptide, non-peptide oligomer or small molecule libraries of compounds (Lam,1997, Anticancer Drug Des.12:145). Combinatorial libraries of test compounds, including small molecule test compounds, can be utilized, and may, for example, be generated as disclosed in Eichler & Houghten, 1995, Mol. Med. Today 1:174-180; Dolle, 1997, Mol. Divers. 2:223-236; and Lam, 1997, Anticancer Drug Des. 12:145-167.

Examples of methods for the synthesis of molecular libraries can be found in the art, for example in: DeWitt *et al.*, 1993, Proc. Natl. Acad. Sci. USA 90:6909; Erb *et al.,* 1994, Proc. Natl. Acad. Sci. USA 91:11422; Zuckermann *et al.*, 1994. J. Med. Chem. 37:2678; Cho *et al.*, 1993, Science 261:1303; Carrell *et al*., 1994, Angew. Chem. Int. Ed. Engl. 33:2059; Carell *et al*., 1994, Angew. Chem. Int. Ed. Engl. 33:2061; and Gallop *et al*., 1994, J. Med. Chem. 37:1233.

Libraries of compounds may be presented from solution (*e.g.,* Houghten, 1992, Bio/Techniques 13:412-421), or on beads (Lam, 1991, Nature 354:82-84), chips (Fodor, 1993, Nature 364:555-556), bacteria (U.S. Patent No. 5,223,409), spores (Patent Nos. 5,571,698; 5,403,484; and 5,223,409), plasmids (Cull *et al*., 1992, Proc. Natl. Acad. Sci. USA 89:1865-1869) or phage (Scott and Smith, 1990, Science 249:386-390; Devlin, 1990, Science 249:404-406; Cwirla *et al.*, 1990, Proc. Natl. Acad. Sci. USA 87:6378-6382; and Felici, 1991, J. Mol. Biol. 222:301-310).

Screening the libraries can be accomplished by any of a variety of commonly known methods. See, *e.g.* , the following references, which disclose screening of peptide libraries: Parmley & Smith, 1989, Adv. Exp. Med. Biol. 251:215-218; Scott & Smith, 1990, Science 249:386-390; Fowlkes *et al.,* 1992; BioTechniques 13:422-427; Oldenburg *et al.,* 1992, Proc. Natl. Acad. Sci. USA 89:5393-5397; Yu *et al*., 1994, Cell 76:933-945; Staudt *et al*., 1988, Science 241:577-580; Bock *et al*., 1992, Nature 355:564-566; Tuerk *et al*., 1992, Proc. Natl. Acad. Sci. USA 89:6988-6992; Ellington *et al*., 1992, Nature 355:850-852; U.S. Patent No. 5,096,815, U.S. Patent No. 5,223,409, and U.S. Patent No. 5,198,346, all to Ladner *et al*., Rebar & Pabo, 1993, Science 263:671-673; and PCT Publication No. WO 94/18318.

Upon identification of a compound that modulates the interaction of SLC (or MIP-3β) with CCR7, such a compound can be further investigated to test for an ability to alter the allergenic or inflammatory response. In particular, for example, the compounds identified via the present methods can be further tested *in vivo* in accepted animal models of SLC (or MIP-3β) related disorders, such as, e.g., allergy, asthma, and inflammation.

Computer modeling and searching technologies permit identification of compounds, or the improvement of already identified compounds, that can modulate the interaction of SLC (or MIP-3β) with CCR7. Having identified such a compound or composition, the binding sites or regions are identified. The binding site can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the binding site by finding where on the target the complexed ligand is found.

Next, the three dimensional geometric structure of the binding site is determined. This can be done by known methods, including X-ray crystallography, which can determine a complete molecular structure. On the other hand, solid or liquid phase NMR can be used to determine certain intra-molecular distances. Any other experimental method of structure determination can be used to obtain partial or complete geometric structures. The geometric structures may be measured with a complexed ligand, natural or artificial, which may increase the accuracy of the binding site structure as determined.

If an incomplete or insufficiently accurate structure is determined, the methods of computer based numerical modeling can be used to complete the structure or improve its accuracy. Any recognized modeling method may be used, including parameterized models specific to particular biopolymers such as proteins or nucleic acids, molecular dynamics models based on computing molecular motions, statistical mechanics models based on thermal ensembles, or combined models. For most types of models, standard molecular force fields, representing the forces between constituent atoms and groups, are necessary, and can be selected from force fields known in physical chemistry. The incomplete or less accurate experimental structures can serve as constraints on the complete and more accurate structures computed by these modeling methods.

Finally, having determined the structure of the binding site, either experimentally, by modeling, or by a combination, candidate modulating compounds can be identified by searching databases containing compounds along with information on their molecular structure. Such a search seeks compounds having structures that match the determined binding site structure and that interact with the groups defining the active site. Such a search can be manual, but is preferably computer assisted. These compounds found from this search are potential CCR7 modulating compounds.

Alternatively, these methods can be used to identify improved modulating compounds from an already known modulating compound or ligand. The composition of the known compound can be modified and the structural effects of modification can be determined using the experimental and computer modeling methods described above applied to the new composition. The altered structure is then compared to the binding site structure of the previously known compound to determine if an improved fit or interaction results. In this manner systematic variations in composition, such as by varying side groups, can be quickly evaluated to obtain modified modulating compounds or ligands of improved specificity or activity.

Further experimental and computer modeling methods useful to identify modulating compounds based upon identification of the binding sites of either CCR7, or SLC (or MIP-3β), will be apparent to those of skill in the art.

Examples of molecular modeling systems are the CHARMm and QUANTA programs (Polygen Corporation, Waltham, MA). CHARMm performs the energy minimization and molecular dynamics functions. QUANTA performs the construction, graphic modelling and analysis of molecular structure. QUANTA allows interactive construction, modification, visualization, and analysis of the behavior of molecules with each other.

A number of articles review computer modeling of drugs interactive with specific proteins, such as Rotivinen *et al.*,) 1988, Acta Pharmaceutical Fennica 97:159-166); Ripka (1988 New Scientist 54-57); McKinaly and Rossmann (1989, Annu. Rev. Pharmacol. Toxiciol. 29:111-122); Perry and Davies, OSAR: Quantitative Structure-Activity Relationships in Drug Design pp. 189-193 Alan R. Liss, Inc. 1989; Lewis and Dean (1989, Proc. R. Soc. Lond. 236:125-140 and 141-162); and, with respect to a model receptor for nucleic acid components, Askew, *et al,.* (1989, J. Am. Chem. Soc. 111:1082-1090). Other computer programs that screen and graphically depict chemicals are available from companies such as BioDesign, Inc. (Pasadena, CA.), Allelix, Inc. (Mississauga, Ontario, Canada), and Hypercube, Inc. (Cambridge, Ontario). Although these are primarily designed for application to drugs specific to particular proteins, they can also be adapted to design of drugs specific to regions of DNA or RNA, once that region is identified.

CCR7 protein, polypeptides and peptide fragments, mutated, truncated or deleted forms of the CCR7 and/or CCR7 fusion proteins can be prepared for a variety of uses, including but not limited to the generation of antibodies, as reagents in diagnostic assays, the identification of other cellular gene products involved in the regulation of SLC (or MIP-3β) related disorders, and as reagents in assays for screening for compounds that can be used as pharmaceutical reagents in the treatment of SLC (or MIP-3β) related disorders. Additionally, based upon information gained from interacting CCR7 and SLC (or MIP-3β), peptide fragments of CCR7 can be derived which inhibit the normal binding of circulating SLC (or MIP-3β) to CCR7 receptors molecules, for *in vivo* therapeutic use.

CCR7 peptides, polypeptides, and fusion proteins can be prepared by recombinant DNA techniques. For example, nucleotide sequences encoding one or more extracellular domains ("ECD") of CCR7 can be synthesized or cloned and ligated together to encode a soluble ECD of the CCR7. The DNA sequence encoding one or more subregions of the ECDs can be ligated together directly or via a linker oligonucleotide that encodes a peptide spacer. Such linkers may encode flexible, glycine-rich amino acid sequences thereby allowing the domains that are strung together to assume a conformation that can bind CCR7 ligands. Alternatively, nucleotide sequences encoding individual domains within the ECD can be used to express CCR7 peptides.

A variety of host-expression vector systems may be utilized to express nucleotide sequences encoding the appropriate regions of CCR7 to produce such polypeptides. Where the resulting peptide or polypeptide is a soluble derivative (*e.g.,* peptides corresponding to the ECDs); and is typically truncated or deleted with respect to transmembrane or cellular domains, the peptide or polypeptide can be recovered from the culture media.

The host-expression vector systems also encompass engineered host cells that express CCR7 or functional equivalents *in situ*, *i.e.*, anchored in the cell membrane. Purification or enrichment of CCR7 from such expression systems can be accomplished using appropriate detergents and lipid micelles and methods well known to those skilled in the art. Additionally, such engineered host cells may themselves be used in situations where it is important not only to retain the structural and functional characteristics of CCR7, but to assess biological activity, *e.g.,* in drug screening assays.

The host-expression vector systems that may be used for purposes of the invention include but are not limited to microorganisms such as bacteria (*e.g., E. coli, B. subtilis*) transformed with recombinant bacteriophage DNA, plasmid DNA or cosmid DNA expression vectors containing CCR7 nucleotide sequences; yeast (*e.g., Saccharomyces, Pichia*) transformed with recombinant yeast expression vectors containing CCR7 nucleotide sequences; insect cell systems infected with recombinant virus expression vectors (*e.g.*, baculovirus) containing CCR7 sequences; plant cell systems infected with recombinant virus expression vectors (*e.g.,* cauliflower mosaic virus, CaMV; tobacco mosaic virus, TMV) or transformed with recombinant plasmid expression vectors *(e.g.,* Ti plasmid) containing CCR7 nucleotide sequences; or mammalian cell systems (*e.g.*, COS, CHO, BHK, 293, 3T3) harboring recombinant expression constructs containing promoters derived from the genome of mammalian cells (*e.g.,* metallothionein promoter) or from mammalian viruses *(e.g.,* the adenovirus late promoter; the vaccinia virus 7.5K promoter).

In bacterial systems, a number of expression vectors may be advantageously selected depending upon the use intended for the CCR7 gene product being expressed. For example, when a large quantity of such a protein is to be produced, for the generation of pharmaceutical compositions of CCR7 protein or for raising antibodies to the CCR7 protein, for example, vectors which direct the expression of high levels of fusion protein products that are readily purified may be desirable. Such vectors include, but are not limited, to the E. coli expression vector pUR278 (Ruther *et al.*, 1983, EMBO J. 2:1791), in which the CCR7 coding sequence may be ligated individually into the vector in frame with the lacZ coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509), and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

Alternatively, any fusion protein may be readily purified by utilizing an antibody specific for the fusion protein being expressed. For example, a system described by Janknecht *et al.* allows for the ready purification of non-denatured fusion proteins expressed in human cell lines (Janknecht *et al*., 1991, Proc. Natl. Acad. Sci. USA 88:8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺•nitriloacetic acid-agarose columns and histidine-tagged proteins are selectively eluted with imidazole-containing buffers.

In an insect system, *Autographa californica* nuclear polyhedrosis virus (AcNPV) is used as a vector to express foreign genes. The virus grows in *Spodoptera frugiperda* cells. The CCR7 coding sequence may be cloned individually into non-essential regions (for example the polyhedrin gene) of the virus and placed under control of an AcNPV promoter (for example the polyhedrin promoter). Successful insertion of a CCR7 gene coding sequence will result in inactivation of the polyhedrin gene and production of non-occluded recombinant virus (*i.e.,* virus lacking the proteinaceous coat coded for by the polyhedrin gene). The recombinant viruses are then used to infect cells in which the inserted gene is expressed (*e.g.,* see Smith *et al*., 1983, J. Virol. 46: 584; Smith, U.S. Patent No. 4,215,051).

In mammalian host cells, a number of viral-based expression systems may be utilized. In cases where an adenovirus is used as an expression vector, the CCR7 nucleotide sequence of interest may be ligated to an adenovirus transcription/translation control complex, *e.g.,* the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing the CCR7 gene product in infected hosts (see, *e.g.*, Logan & Shenk, 1984, Proc. Natl. Acad. Sci. USA 81:3655-3659). Specific initiation signals may also be required for efficient translation of inserted CCR7 nucleotide sequences. These signals include the ATG initiation codon and adjacent sequences. In cases where an entire CCR7 gene or cDNA, including its own initiation codon and adjacent sequences, is inserted into the appropriate expression vector, no additional translational control signals may be needed. However, in cases where only a portion of the CCR7 coding sequence is inserted, exogenous translational control signals, including, perhaps, the ATG initiation codon, must be provided. These exogenous translational control signals and initiation codons can be of a variety of origins, both natural and synthetic. The efficiency of expression may be enhanced by the inclusion of appropriate transcription enhancer elements, transcription terminators, etc (see Bittner *et al.*, 1987, Methods in Enzymol. 153:516-544).

In addition, a host cell strain may be chosen which modulates the expression of the inserted sequences, or modifies and processes the gene product in the specific fashion desired. Such modifications (*e.g.,* glycosylation) and processing (*e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. Accordingly, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3 and WI38 cell lines.

For long-term, high-yield production of recombinant proteins, stable expression is preferred. For example, cell lines which stably express the CCR7 sequences described above may be engineered. Rather than using expression vectors which contain viral origins of replication, host cells can be transformed with DNA controlled by appropriate expression control elements (*e.g.,* promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and may also facilitate isolation of cells that stably integrate the plasmid into their chromosomes, and which in turn can be cloned and expanded into cell lines. This method may advantageously be used to engineer cell lines which express the CCR7 gene product. Such engineered cell lines may be particularly useful in screening and evaluation of compounds that affect the endogenous activity of the CCR7 gene product.

A number of selection systems may be used, including but not limited to the herpes simplex virus thymidine kinase (Wigler *et al*., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy *et al*., 1980, Cell 22:817) genes can be employed in tk⁻, hgprt⁻ or aprt⁻ cells, respectively. Also, antimetabolite resistance can be used as the basis of selection for the following genes: dhfr, which confers resistance to methotrexate (Wigler *et al.*, 1980, Natl. Acad. Sci. USA 77:3567; O'Hare, *et al*., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin *et al.*, 1981, J. Mol. Biol. 150:1); and hygro, which confers resistance to hygromycin (Santerre *et al.*, 1984, Gene 30:147).

Antibodies that specifically recognize one or more epitopes of CCR7, or epitopes of conserved variants of CCR7, or peptide fragments of CCR7 are also encompassed by the invention. Such antibodies include but are not limited to polyclonal antibodies, monoclonal antibodies (mAbs), humanized or chimeric antibodies, single chain antibodies, Fab fragments, F(ab')₂ fragments, fragments produced by a Fab expression library, anti-idiotypic (anti-Id) antibodies, and epitope-binding fragments of any of the above.

The antibodies of the invention may be used, for example, in the detection of CCR7 in a biological sample and may, therefore, be utilized as part of a diagnostic technique whereby patients may be tested for abnormal amounts of CCR7. Antibodies that specifically recognize mutant forms of CCR7, may be particularly useful as part of a diagnostic technique. Such antibodies may also be utilized in conjunction with, for example, compound screening schemes, as described, above, for the evaluation of the effect of test compounds on expression and/or activity of the CCR7 gene product. Additionally, such antibodies can be used in conjunction with the gene therapy techniques described, below, *e.g*., to evaluate the normal and/or engineered CCR7-expressing cells prior to their introduction into the patient. Such antibodies may additionally be used as a method for the inhibition of abnormal CCR7 activity. Thus, such antibodies may, therefore, be utilized as part of SLC (or MIP-3β)-related disorder treatment methods.

For the production of antibodies, various host animals may be immunized by injection with CCR7, a CCR7 peptide (*e.g.,* one corresponding the a functional domain of the receptor), truncated CCR7 polypeptides (CCR7 in which one or more domains have been deleted), functional equivalents of CCR7 or mutants of CCR7. Such host animals may include but are not limited to rabbits, mice, hamsters and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.* Polyclonal antibodies are heterogeneous populations of antibody molecules derived from the sera of the immunized animals.

Monoclonal antibodies, which are homogeneous populations of identical antibodies to a particular antigen, may be obtained by any technique which provides for the production of antibody molecules by continuous cell lines in culture. These include, but are not limited to, the hybridoma technique of Kohler and Milstein, (1975, Nature 256:495-497 and U.S. Patent No. 4,376,110), the human B-cell hybridoma technique (Kosbor *et al.*, 1983, Immunology Today 4:72; Cole *et al*., 1983, Proc. Natl. Acad. Sci. USA 80:2026-2030), and the EBV-hybridoma technique (Cole *et al.,* 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

In addition, techniques developed for the production of "chimeric antibodies" (Morrison *et al*., 1984, Proc. Natl. Acad. Sci., 81:6851-6855; Neuberger *et al*., 1984, Nature, 312:604-608; Takeda *et al.,* 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region.

Alternatively, techniques described for the production of single chain antibodies (U.S. Patent 4,946,778; Bird, 1988, Science 242:423-426; Huston *et al.,* 1988, Proc. Natl. Acad. Sci. USA 85:5879-5883; and Ward *et al.,* 1989, Nature 334:544-546) can be adapted to produce single chain antibodies against CCR7 gene products. Single chain antibodies are formed by linking the heavy and light chain fragments of the Fv region via an amino acid bridge, resulting in a single chain polypeptide.

Antibody fragments which recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse *et al.*, 1989, Science, 246:1275-1281) to allow rapid and easy identification of monoclonal Fab fragments with the desired specificity.

Antibodies to CCR7 can, in turn, be utilized to generate anti-idiotype antibodies that "mimic" CCR7, using techniques well known to those skilled in the art (see, *e.g.,* Greenspan & Bona, 1993, FASEB J 7(5):437-444; and Nissinoff, 1991, J. Immunol. 147(8):2429-2438). For example antibodies which bind to the CCR7 extracellular domain ("ECD"), and competitively inhibit the binding of ligand to the CCR, can be used to generate anti-idiotypes that "mimic" the ECD and, therefore, bind and neutralize ligand. Such neutralizing anti-idiotypes or Fab fragments of such anti-idiotypes can be used in therapeutic regimens to neutralize the native ligand and treat CCR7-related disorders, such as allergenic disorders and asthma, all as before.

Alternatively, antibodies to CCR7 that can act as agonists of CCR7 activity can be generated. Such antibodies will bind to the CCR7 and activate the signal transducing activity of the receptor. In addition, antibodies that act as antagonist of CCR7 activity, i.e. inhibit the activation of CCR7 receptor would be particularly useful for treating SLC (or MIP-3β) related disorders, again such as allergenic disorders, asthma, and inflammatory disorders.

Genetically engineered cells that express soluble CCR7 ECDs or fusion proteins e.g. fusion Ig molecules can be administered *in vivo* where they may function as "bioreactors" that deliver a supply of the soluble molecules. Such soluble CCR7 polypeptides and fusion proteins, when expressed at appropriate concentrations, should neutralize or "mop up" the native ligand for CCR7, and thus act as inhibitors of CCR7 activity and may therefore be used to treat SLC (or MIP-3β)-related disorders, such as allergenic disorders, asthma, and inflammatory disorders, all as before.

The immunoassays which can be used include but are not limited to competitive and non-competitive assay systems using techniques such as western blots, immunohisto-chemistry radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few.

In additional embodiments, diseases and disorders involving decreased immune responsiveness during an infection can be diagnosed, or their suspected presence can be screened for, or a predisposition to develop such disorders can be detected, by detecting decreased levels of CCR7 protein, CCR7 RNA, or CCR7 functional activity (*e.g.,* binding to SLC, anti-CCR7 antibody-binding activity etc.), or by detecting mutations in CCR7 RNA, DNA or CCR7 protein (*e.g*., translocations in CCR7 nucleic acids, truncations in CCR7 gene or protein, changes in nucleotide or amino acid sequence relative to wild-type CCR7) that cause decreased expression or activity of CCR7. Such diseases and disorders include but are not limited to allergic and asthmatic disorders (*e.g.,* allergic rhinitis, allergic asthma, bronchoconstriction, inflammatory disorders, graft rejection, and autoimmune diseases.

By way of example, levels of CCR7 can be detected by immunoassay, levels of CCR7 RNA can be detected by hybridization assays (*e.g.,* Northern blots, *in situ*hybridization), and CCR7 activity can be assayed by measuring binding activities *in vivo* or *in vitro.* Translocations, deletions, and point mutations in CCR7 nucleic acids can be detected by Southern blotting, FISH, RFLP analysis, SSCP, PCR using primers that preferably generate a fragment spanning at least most of the CCR7 gene, sequencing of CCR7 genomic DNA or cDNA obtained from the patient, etc.

In a preferred embodiment, levels of CCR7 mRNA or protein in a patient sample are detected or measured relative to the levels present in an analogous sample from a subject not having the SLC or MIP-3β-related disorder, such as an allergenic disorder, asthma, or inflammatory disorder. Decreased levels indicate that the subject may develop, or have a predisposition to developing, an allergenic disorder, asthma, an inflammatory disorder, or other conditions named above.

In a specific embodiment, levels of mRNA or protein in a patient sample are detected or measured, relative to the levels present in an analogous sample from a subject not having the disorder, in which increased levels indicate that the subject has, or has a predisposition to, an autoimmune disorder.

Kits for diagnostic use are also provided, that comprise in one or more containers an anti-CCR7 antibody, and, optionally, a labeled binding partner to the antibody. Alternatively, the anti-CCR7 antibody can be labeled (with a detectable marker, *e.g.*, a chemiluminescent, enzymatic, fluorescent, or radioactive moiety). A kit is also provided that comprises in one or more containers a nucleic acid probe capable of hybridizing to CCR7 RNA. In a specific embodiment, a kit can comprise in one or more containers a pair of primers (*e.g*., each in the size range of 6-30 nucleotides) that are capable of priming amplification [*e.g*., by polymerase chain reaction (see *e.g.,* Innis *et al*., 1990, PCR Protocols, Academic Press, Inc., San Diego, CA), ligase chain reaction (see EP 320,308) use of Qβ replicase, cyclic probe reaction, or other methods known in the art] under appropriate reaction conditions of at least a portion of a CCR7 nucleic acid. A kit can optionally further comprise in a container a predetermined amount of a purified SLC or MIP-3β, or CCR7 nucleic acid, protein, derivative, analog, or fragment thereof, or, *e.g.*, for use as a standard or control.

The invention encompasses methods and compositions for modifying the interaction with SLC or MIP-3β and CCR7, and for treating SLC or MIP-3β related disorders, including, but not limited to, allergy, asthma, and inflammation. Because a loss of normal CCR7 gene product function may result in the development of a SLC or MIP-3β-related disorder phenotype, an increase in CCR7 gene product activity, or activation of the CCR7 pathway (*e.g.*, downstream activation) would facilitate progress towards a normal SLC or MIP-3β-related state in individuals exhibiting a deficient level of CCR7 gene expression and/or CCR7 activity.

Alternatively, symptoms of certain SLC or MIP-3β related disorders may be ameliorated by decreasing the level of CCR7 gene expression, and/or CCR7 gene activity, and/or down-regulating activity of the CCR7 pathway (*e.g.*, by targeting downstream signaling events).

A CCR7 antagonist can be used to treat conditions such as asthma or inflammation. Agonists of CCR7 can be used to stimulate CCR7 activity, in mammals in need of such treatment.

Toxicity and therapeutic efficacy of such compounds can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (*i.e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

### Pharmaceutical compositions

Pharmaceutical compositions for use in accordance with the present invention may be formulated in conventional manner using one or more physiologically acceptable carriers or excipients.

Thus, the compounds and their physiologically acceptable salts, hydrates and solvates may be formulated for administration by inhalation or insufflation (either through the mouth or the nose) or oral, buccal, parenteral or rectal administration.

For oral administration, the pharmaceutical compositions may take the form of, for example, tablets or capsules prepared by conventional means with pharmaceutically acceptable excipients such as binding agents (*e.g.,* pregelatinised maize starch, polyvinylpyrrolidone or hydroxypropyl methylcellulose); fillers (*e.g.,* lactose, microcrystalline cellulose or calcium hydrogen phosphate); lubricants (*e.g.,* magnesium stearate, talc or silica); disintegrants (*e.g*., potato starch or sodium starch glycolate); or wetting agents (*e.g.,* sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g.,* sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, *e.g.,* by bolus injection or continuous infusion. Formulations for injection may be presented in unit dosage form, *e.g.,* in ampules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g.,* sterile pyrogen-free water, before use.

The compounds may also be formulated in rectal compositions such as suppositories or retention enemas, *e.g.,* containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### Example 1 Binding Assay

Membrane preparation: HUT-78 cells were cultured in RPMI media with 10% FBS, pen-strep (100 u/ml), L-glutamine (2 mM), HEPES (10 mM) and non-essential amino acids (0.1 mM). The cells were collected by centrifugation at 170xg for 5 min and resuspended to 50 x 10^6 cells/ml in Dulbeccoco's Ca²⁺ and Mg²⁺ free media containing 8 mg/l aprotinin at 4°C. The cells were then disrupted with a Polytron homogenizer and centrifuged at 30,000xg for 10 min. The pellet was washed 6 times and stored at -80°C.

Binding assay: The binding assay was run in a buffer containing HEPES (50 mM), CaCl₂ (1 mM), MgCl₂ (5 mM), NaCI (150 mM) and BSA (0.5 mg/ml), pH 7.2. The pellet was resuspended to 8x10⁶ cell equivalents/ml and 50 µl was added to microtiter plate wells containing 130 µl of buffer with various concentrations of compounds, and 0.5 mg PEI-treated wheatgerm agglutinin-coated scintillation proximity beads (Amersham). The binding was initiated by the addition of 20 µl of 600 pM ¹²⁵I-MIP-3β (New England Nuclear). The microtiter plate was mixed and then the beads were allowed to settle overnight before counting in a Microbeta plus liquid scintillation counter (Wallac).

The binding assay as adaptable to high throughput screening methods as recognized in the art.

### Example 2 In Vitro Chemotaxis Assay

Lymphocyte and PHA blast chemotaxis to appropriate chemokine was measured using a 48-well Boyden chamber (Neuro Probe Inc., Cabin John, MD). Agonists were diluted in RPMI media (BioWhittaker, Walkersville, MD) containing 0.1% bovine serum albumin (Invitrogen, Carlsbad, CA) and added to the bottom wells of the chemotaxis chamber. Peripheral mononuclear blood cells were resuspended in RPMI/BSA media at a concentration of 2.5 x 10⁶ cells per ml. Fifty µl of this cell suspension was then added to the upper chamber. A 5µm PVP-free polycarbonate filter, coated on the bottom with 10 microgram/ml of human type IV collagen (Neuro Probe Inc.), was used to separate the wells of the chamber

Chambers were incubated for 60 minutes in a 5% CO₂-humidified atmosphere at 37°C. After the incubation period, the filters were removed, the top side was scraped to remove non-migrating cells, and the filters were stained with Diff-Quik stain (Dade Behring AG, Dudingen, Switzerland). The number of PMBC cells migrating was counted by light microscopy. The mean of three high powered fields was then determined. The number of migrating PMBC cells was calculated by subtracting this number from the number of cells per high powered field in those wells not containing any agonist.

### Example 3

The following compound was identified as inhibiting the binding of MIP-3β to CCR7. In the binding assay of Example 1, it gave an IC₅₀ value of 1.5µM.

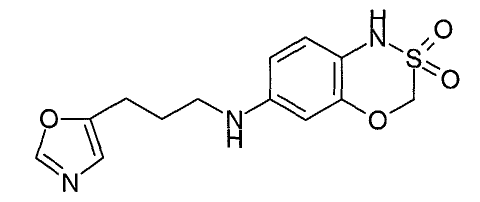

### Example 4

The following compound was also identified as inhibiting the binding of MIP-3β to CCR7. In the binding assay of Example 1, it gave an IC₅₀ value of 2.2 µM.

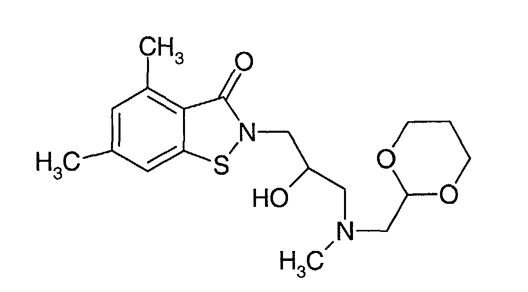

### Example 5 Calcium Mobilization Asssay

T cell blasts were harvested in flux buffer (1X Hanks, 10 mM Hepes, 1.6mM CaCl₂, pH 7.3), loaded with INDO-1-AM (20µg/ml) for 30 min at 37°C, and rewashed in flux buffer. Cells (0.25x 10⁶/ml of flux buffer) were placed in a continuously stirred cuvette at 37°C in a fluorimager (Photon Technology Inc., South Brunswick, NJ). Cells were stimulated with SLC or MIP-3 beta and the calcium-related fluorescence changes were recorded. The intracellular concentration of Ca⁺² ion was then determined.

## Claims

1. A method for detecting if a test compound affects the binding of secondary lymphoid chemokine (SLC) or macrophage inflammatory protein-3β (MIP-3β) to CCR7 receptor comprising:
(a) contacting a sample of CCR7 with SLC or MIP-3β and measuring the binding of SLC or MIP-3β thereto;
(b) contacting a similar sample of CCR7 with SLC or MIP-3β and also an amount of said compound, and measuring the binding of said SLC or MIP-3β to said CCR7; and
(c) comparing the results in (a) and (b) to determine if the binding of SLC or MIP-3β is affected by the presence of said compound.

2. A method for determining if a test compound affects an activity of CCR7 receptor that is normally dependent upon the binding of SLC or MIP-3β thereto, comprising the steps of:
(a) contacting a sample of CCR7 with SLC or MIP-3β and measuring a resultant activity of said CCR7;
(b) contacting a similar sample of CCR7 with SLC or MIP-3β in the presence of an amount of said compound, and measuring said resultant activity of CCR7; and
(c) comparing the results in (a) and (b) to determine if the SLC dependent or MIP-3β dependent activity of CCR7 is affected by the presence of said compound.

3. The method of Claim 2, in which the test compound is identified as an antagonist that interferes with an SLC dependent or MIP-3β dependent activity of CCR7.

4. The method of Claim 2, wherein the test compound is an antibody, or antibody fragment, specific for CCR7.

5. The method of Claim 2, wherein the test compound is an antibody, or antibody fragment, specific for SLC or MIP-3β.

6. The method of Claim 2, wherein the test compound is an agonist that enhances an SLC dependent or MIP-3β dependent activity of CCR7.

7. The method of Claim 2 wherein the SLC dependent or MIP-3β dependent activity of CCR7 contributes to a disease state that is selected from the group consisting of: an autoimmune disease, an inflammatory disease, an allergic disease, transplant rejection, reperfusion injury, atherosclerosis, restinosis, enhanced HIV infectivity mediated by co-receptor usage, a granulomatous disease, inflammation-associated infection, and metastasis of cancer cells.

8. The method of claim 7, wherein said automimmune disease is selected from the group consisting of rheumatoid arthritis, type I diabetes, lupus, inflammatory bowel disease, primary sclerosing cholangitis, optic neuritis, psoriasis, multiple sclerosis, polymyalgia rheumatica, uveitis, and vasculitis.

9. The method of claim 7, wherein said inflammatory disease is selected from the group consisting of osteoarthritis, adult respiratory distress syndrome, respiratory distress syndrome of infancy, ischemia reperfusion injury, and glomerulonephritis.

10. The method of claim 7, wherein said allergic disease is selected from the group consisting of asthma, allergic rhinitis, and atopic dermatitis.

11. The method of claim 7, wherein said rejected transplant is selected from the group consisting of an organ or tissue transplant, or transplant of cells, whether rejection thereof is chronic or acute.

12. The method of claim 7 wherein said rejected transplant is a xenotransplant.

13. The method of claim 7, wherein said granulomatous disease is selected from the group consisting of sarcoidosis, leprosy, and tuberculosis.

14. The method of claim 7, wherein said inflammation-associated infection is selected from the group consisting of hepatitis, influenza and infection with Guillan-Barre virus.

15. The method of Claim 2, wherein the CCR7 activity measured is the ability to bind to SLC or MIP-3β.

16. The method of claim 2, wherein said CCR7 is present on the surface of a mammalian cell, a membrane fragment thereof, or a lipid vesicle.

17. The method of Claim 16, wherein the CCR7 receptor is present on the surface of a mammalian cell, and the SLC dependent activity of said cell, mediated by CCR7, is selected from the group consisting of migration to an inflammatory site, migration to a site of antigen presentation, cell activation, cell proliferation, and secretion of a cytokine.

18. The method of Claim 16, wherein the CCR7 receptor is present on the surface of a mammalian cell, and the MIP-3β dependent activity of said cell, mediated by CCR7, is selected from the group consisting of migration to an inflammatory site, migration to a site of antigen presentation, cell activation, cell proliferation, and secretion of a cytokine.

19. The method of Claim 16, wherein the CCR7 receptor is present on the surface of a mammalian cell, and the SLC dependent or MIP-3β dependent activity of CCR7 is an effect on said cell, selected from the group consisting of migration to an inflammatory site, migration to a site of antigen presentation, cell activation, cell proliferation, and secretion of a cytokine.

20. A method for diagnosing a disorder in a patient that is mediated by the interaction of CCR7 receptor and SLC or MIP-3β, comprising the steps of:
(a) measuring an SLC dependent or MIP-3β dependent activity of CCR7 receptor in a patient sample; and
(b) comparing said measurement to that determined from clinically normal individuals.

21. A diagnostic kit, packaged in a container, comprising:
(a) SLC, or a fragment thereof; and
(b) a reagent selected from:
(b₁) CCR7 protein or a fragment thereof,
(b₂) nucleic acid encoding a CCR7 protein or a fragment thereof, and
(b₃) cells expressing CCR7.

22. A diagnostic kit, packaged in a container, comprising:
(a) MIP-3β, or a fragment thereof; and
(b) a reagent selected from:
(b₁) CCR7 protein or a fragment thereof,
(b₂) nucleic acid encoding a CCR7 protein or a fragment thereof, and
(b₃) cells expressing CCR7.

23. The method of Claim 2 wherein the SLC dependent or MIP-3β dependent activity of CCR7 contributes to a disease state that is selected from the group consisting of chronic bronchitis, atherosclerosis, restinosis, and enhanced HIV infectivity mediated by co-receptor usage.
